**Europäisches Patentamt**

**European Patent Office**

**Office européen des brevets**

(11) Veröffentlichungsnummer: **0 002 811**
**B2**

(12) **NEUE EUROPÄISCHE PATENTSCHRIFT**

(45) Veröffentlichungstag der neuen Patentschrift:
**13.07.88**

(21) Anmeldenummer: **78101805.6**

(22) Anmeldetag: **21.12.78**

(51) Int. Cl.⁴: **A 61 N 1/32, H 03 B 21/02**

(54) **Vorrichtung zur Interferenzstromtherapie.**

(30) Priorität: **27.12.77 AT 9305/77**

(43) Veröffentlichungstag der Anmeldung:
**11.07.79 Patentblatt 79/14**

(45) Bekanntmachung des Hinweises auf die Patenterteilung:
**24.02.82 Patentblatt 82/8**

(45) Bekanntmachung des Hinweises auf die Entscheidung
über den Einspruch:
**13.07.88 Patentblatt 88/28**

(84) Benannte Vertragsstaaten:
**BE CH DE FR GB IT NL SE**

(56) Entgegenhaltungen:
**DE - A - 2 419 768**
**DE - A - 2 615 157**
**DE - A - 2 632 700**
**DE - B - 1 298 557**
**DE - B - 2 159 437**
**GB - A - 1 502 432**
**US - A - 3 588 732**
**US - A - 3 746 891**
**US - A - 3 895 639**
**US - A - 4 071 033**

(73) Patentinhaber: **Somartec SA, 17 Bd. Helvetique,**
**CH-1311 Genf (CH)**

(72) Erfinder: **Rodler, Hans, Bahnhofplatz 3,**
**D-8228 Freilassing (DE)**

(74) Vertreter: **Lichti, Heiner, Dipl.-Ing. et al, Patentanwälte**
**Dr. Ing. Hans Lichti Dipl.-Ing. Heiner Lichti Dipl.-Phys.**
**Dr.rer.nat. Jost Lempert Durlacher**
**Strasse 31 Postfach 410760, D-7500 Karlsruhe 41 (DE)**

## Beschreibung

Die Erfindung betrifft eine Vorrichtung zur Interferenzstromtherapie, mit einem gemeinsamen Oszillator für beide vorhandenen Stromkreise.

Bei den bisher bekannten Anordnungen DL-PS 1 109 280 (Nemec), OE-PS 203 147 (Nemec) und OE-PS 191 082 (Nemec) wurden zwei Frequenzen mit ca. 5000 Hz, wobei die eine geringfügig von der zweiten differiert, z.B. 5000 und 5001 Hz, verwendet. Die grosse Schwierigkeit bei diesen Anordnungen besteht darin, dass diese geringen Frequenzunterschiede sehr schwer stabil gehalten werden können, zumal die eine der beiden Frequenzen veränderlich sein muss.

Bei einer Vorrichtung nach der US-A 3 895 639 wird von einem gemeinsamen Oszillator für beide Stromkreise ausgegangen, der eine Frequenz im Anwendungsbereich von wenigen Kiloherz erzeugt. Zur Erzeugung einer Schwebungsfrequenz im Überlagerungsgebiet im zu behandelnden menschlichen Körper wird in einem der beiden Zweige mittels eines Phasenschiebers eine Verschiebung der Phase eines der A.C.-Ströme hervorgerufen.

Nachteilig bei dieser Vorgehensweise ist, dass zur erforderlichen rhythmischen Veränderung der Phasenlage der notwendige Funktionsgenerator eine äusserst stabile Amplitude aufweisen muss. Dies erfordert einen aufwendigen und teuren Aufbau der Vorrichtung nach der US-A 3 895 639. Die Endphasenlagen bei 180° und 0° müssen nämlich äusserst exakt eingestellt werden, da sonst verzerrte Interferenzschwingungen aufgrund ungenauer Nulldurchgänge entstehen würden. Bei der bekannten Vorrichtung ist also mit anderen Worten eine dauernde, kontrollierte Verschiebung der Phasenlage derart notwendig, dass eine feste Frequenzänderung erzielt wird und nicht aufgrund von Unstabilitäten die Frequenz am Ausgang schwankt, wodurch sich dann keine stabile Schwebung ergeben würde. Um aber hier die erforderliche Stabilität und Genauigkeit zu erreichen sind aufwendige Massnahmen notwendig, mit denen danach aber keine vollständig befriedigende Stabilität insbesondere bei geringen gewünschten Frequenzunterschieden erzielbar ist. Aus der US-A 3 746 891 ist bei einem nicht gattungsgemässen Sinuswellengenerator eine Rückkopplung des Ausgangs eines Teilers auf den gleichen Teiler bekannt.

Der Erfindung liegt die Aufgabe zugrunde, eine Vorrichtung zur Interferenzstromtherapie zu schaffen, mittels der in einfacher Weise ein stabiler Frequenzunterschied in beiden Stromkreisen, insbesondere bei kleinen Frequenzunterschieden erzielbar ist.

Erfindungsgemäss wird die genannte Aufgabe dadurch gelöst, dass der Oszillator ein Hochfrequenzoszillator ist; dass der Hochfrequenzoszillator mit den Eingängen mindestens zweier digitaler Frequenzteiler verbunden ist; dass zumindest an einem Frequenzteiler eine digitale Rückkopplungseinrichtung zum Einstellen eines geringen Frequenzunterschiedes der an den Ausgängen der Frequenzteiler gegebenen Behandlungsströme vorgesehen ist.

Mit anderen Worten wird also zunächst erfindungsgemäss ein Hochfrequenzgenerator, der im 10 MHz-Bereich arbeitet, vorgesehen. Weiterhin wird erfindungsgemäss die vorgegebene Hochfrequenz zwei oder mehr parallel angeordneten digitalen Frequenzteilern zugeführt, um die Hochfrequenz auf den Behandlungsfrequenzbereich in einem vorgegebenen Grundfrequenzverhältnis herunterzuteilen. Darüber hinaus ist vorgesehen, dass die Frequenz des einen Stromkreises durch eine digitale Rückkopplungseinrichtung in veränderbarer Weise einstellbar ist.

Während bei der US-A 3 895 639 eine kontinuierliche Phasenänderung oder eine dauernde Veränderung des Phasenunterschiedes vorgesehen wird, um zu einem festen Frequenzunterschied in beiden Stromkreisen und damit im Körper zu einer Schwebung mit einer festen Frequenz zu gelangen, ergibt sich durch die erfindungsgemäss vorgenommene Frequenzteilung in beiden Stromkreisen ein fester Frequenzunterschied und damit eine feste Schwebungsfrequenz im Körper des zu behandelnden Patienten zwangsläufig.

Bei der erfindungsgemässen Vorrichtung werden also auf digitalem Wege die beiden unterschiedlichen Frequenzen durch Teilung gewonnen. Dadurch, dass zumindest einer der Frequenzteiler veränderlich ist, können beliebige Frequenzdifferenzen eingestellt werden. Wenn t1 das Teilerverhältnis des einen Frequenzteilers und t2 das Teilerverhältnis des zweiten Frequenzteilers, fi die Interferenzfrequenz und fh die Frequenz des Hochfrequenzoszillators ist, dann errechnet sich die Interferenzfrequenz nach folgender Formel:

$$fi = \frac{fh}{t1} - \frac{fh}{t2}$$

Bei einem Frequenzteilerverhältnis t1 von 10.000 ist die zur Behandlung dienende Interferenz bei einer Hochfrequenz von 50 MHz, wenn das zweite Frequenzteilerverhältnis t2 10.001 ist, 0,5 Hz und die dem Körper aufgedrückte Mittelfrequenz 5000 Hz. Durch Änderung des zweiten Frequenzteilerverhältnisses auf 10.002 ist die Ausgangsfrequenz des zweiten Frequenzteilers 4.999 Hz und die Interferenzfrequenz damit 1 Hz. Durch Umprogrammieren der Teiler mittels der zugeschalteten Vorrichtung können also unterschiedliche Frequenzdifferenzen und damit unterschiedliche, im Körper auftretende Interferenzfrequenzen erreicht werden.

Eine besonders zweckmässige Weiterentwicklung der Erfindung besteht darin, dass die digitale Rückkopplungseinrichtung aus einer zwischen den Hochfrequenzoszillator und den Eingang des Frequenzteilers geschalteten, elektronisch schaltbaren Sperrvorrichtung, die mittels einer Steuereinrichtung mit dem Ausgang des Frequenzteilers verbunden ist, besteht und pro Ausgangsperiode eine Anzahl Taktimpulse aussperrt und damit die Ausgangsfrequenz des Frequenzteilers erniedrigt. Die Frequenzänderung des einen Frequenzteilers wird also durch Aussperrung einzelner oder mehrerer Impulse der vom Hochfrequenzoszillator gelieferten Taktimpulse pro Periode erreicht. Die Periode des Ausgangssignals des Frequenzteilers wird durch die Anzahl der ausgelassenen Taktimpulse verlängert. Die Anzahl der gesperrten Taktimpulse kann hierbei durch einen Zähler oder ein Zeitglied digital bestimmt werden. Die Formel für die Frequenzänderung, wenn fi für Interferenzfrequenz, fh für die Frequenz des Hochfrequenzoszillators und t für das Teilerverhältnis steht, ist:

$$fi = \frac{fh}{t} - \frac{fh - \dfrac{fh}{x\,t}}{t}$$

x ist der Anteil der ausgesperrten Impulse.

Für 50 MHz Hochfrequenz ist bei einem Teilerverhältnis von 10.000 daher die kleinstmögliche Interferenzfrequenz 0,5 Hz.

Zur Verkleinerung der Hochfrequenz wird vorgeschlagen, dass die digitale Rückkopplungseinrichtung aus einer Modulatorschaltung besteht, die zwischen dem Hochfrequenzoszillator mittels eines Phasenschiebers und dem Eingang des Frequenzteilers geschaltet ist, sowie dass eine Steuerschaltung mit dem Ausgang des Frequenzteilers verbunden ist, die bewirkt, dass pro Ausgangsperiode in der durch die Steuerschaltung festgelegten Zeit phasenverschobene Impulse zwischen den Hochfrequenzoszillatorimpulsen hinzugefügt werden und damit die Ausgangsfrequenz am Frequenzteiler erhöht wird.

Gemäss einer bevorzugten Weiterbildung ist vorgesehen, dass die Steuerschaltung aus einem monostabilen Multivibrator mit steuerbarer Impulsbreite besteht.

De Frequenzänderung am Ausgang des Frequenzteilers wird in diesem Falle durch Einfügen zusätzlicher Impulse zu den Taktimpulsen in Abhängigkeit von der Ausgangsperiode erreicht, wobei die Anzahl der hinzugefügten Impulse durch einen monostabilen Multivibrator mit einstellbarer Impulsbreite oder durch einen Zähler erzielt werden kann, wobei die zusätzlichen Impulse zu den Taktimpulsen phasenverschoben sind und in den Pausen der Taktimpulse eingefügt werden. Die Periodendauer der Ausgangsperiode wird dadurch verkürzt. Die Formel hierfür lautet:

$$fi = \frac{fh + x\,\dfrac{\cdot fh}{t}}{t} - \frac{fh}{t}$$

In besonders bevorzugter Weiterbildung ist vorgesehen, dass einem Frequenzteiler ein weiterer Frequenzteiler nachgeschaltet ist, durch dessen mit Hilfe eines Umschalters erzeugten Ausgangssignals über eine Steuerschaltung das Teilerverhältnis des veränderbaren Frequenzteilers automatisch kontinuierlich veränderbar ist.

Da die Interferenzfrequenz nicht nur stabil eingestellt werden soll, sondern auch automatisch in einem bestimmten Rhythmus variiert werden soll, ist also dem Frequenzteilerausgang ein weiterer Frequenzteiler nachgeschaltet, dessen Ausgang mit Hilfe einer Schalteinrichtung eine Steuerschaltung zur Veränderung eines in einem Kreis vorgesehenen Frequenzteilers beeinflusst. Durch diese Massnahme wird der veränderliche Frequenzteiler verschiedene Teilerverhältnisse durchlaufen, so dass im Körper automatisch hintereinander in beliebiger Reihenfolge verschiedene Interferenzfrequenzen auftreten. Es sind somit nicht nur fest einstellbare Frequenzen, sondern auch automatisch ablaufende Programme mit dieser Einrichtung erstellbar.

Diese Weiterbildung erlaubt es also, die Differenzfrequenz zunächst automatisch einzustellen und im weiteren die Differenzfrequenz automatisch nach einem vorgegebenen Programm zu verändern. Dazu ist eben der weitere Frequenzteiler an den Ausgang eines der beiden erläuterten Frequenzteiler angeschlossen. Der zusätzliche Frequenzteiler steuert mit seinem Ausgangssignal eine Steuerschaltung, durch die das Teilungsergebnis gesteuert wird. Der zusätzliche Frequenzteiler wird dabei seinerseits durch eine weitere Einrichtung derart verändert werden, dass das Programm, nach dem die Differenzfrequenz verändert wird, noch in verschiedenartiger Weise variiert werden kann.

Bei den Frequenzteilern, bei denen das Impuls-Pausenverhältnis nicht 1:1 ist, ist es zweckmässig, diesen eine Impulsformerstufe nachzuschalten, die aus den Ausgangsimpulsen eine symmetrische Rechteckwelle erzeugt. Da bei der Behandlung mit Interferenzfrequenzen sinoide Stromformen gebräuchlich sind, ist in Weiterbildung jedem Frequenzteiler zur Umwandlung der Rechteckkurve in eine Sinuskurve eine Kurvenformstufe nachgeschaltet.

Eine volldigitalisierte Ausbildung der Erfindung sieht vor, dass die digitale Rückkopplungseinrichtung einen Zähler aufweist, dessen Takteingang mit dem Hochfrequenzoszillator verbunden ist, während die Ausgänge des Zählers hingegen mit Vergleichseingängen A eines Digitalkomparators und ein Teil der Vergleichseingänge B mit den Ausgängen eines Vor-Rückwärtszählers verbunden sind, dessen Taktfrequenz durch Einzelimpulse automatisch getaktet wird; dass ein Teil der Vergleichseingänge B des Komparators mit einer logischen «1» verbunden ist, wobei der Ausgang A-B des Komparators zur Abnahme der geregelten Frequenz mit einer Impulsformerstufe und dem Reseteingang des Zählers verbunden ist.

Der zur Frequenzuntersetzung dienende Zähler zählt die Impulse des Hochfrequenzoszillators bis seine Ausgänge mit den Ausgängen des Vor-Rückwärtszählers und der fest beschalteten Vergleichseingänge B übereinstimmen. Ist dieser Zustand erreicht, gibt der Komparator am Ausgang A-B einen Impuls ab und stellt den Untersetzerzähler durch den Reseteingang auf Null zurück und schaltet gleichzeitig die Impulsformerstufe um. Der Zähler beginnt neu zu zählen, wobei sich die Vorgänge wiederholen und der Ausgang einer Impulsfolge entsprechend der fest geschalteten Vergleichseingänge B des Komparators und der Ausgänge des Vor-Rückwärtszählers abgibt. Die feste Beschaltung der Komparatoreingänge B bewirkt also die Grundteilung, und jede Erhöhung des Vor-Rückwärtszählers bewirkt eine Vergrösserung der Ausgangsperiode um eine Taktperiode, so dass eine sehr feinstufige Frequenzabnahme entsteht. Die Grösse der Frequenzabnahme kann daher durch einen Takt am Vor-Rückwärtszähler verändert werden, wodurch dieser einen neuen Zustand annimmt.

Erfindungsgemäss kann weiter vorgesehen sein, dass der Hochfrequenzoszillator, die Frequenzteiler und die digitale Rückkopplungseinrichtung einen entsprechenden Mikroprozessor aufweisen, der im Sinne der Funktionsabläufe programmierbar ist. Sämtliche beschriebenen Funktionen können durch die Software des Mikroprozessors dargestellt werden und daher im Sinne der Erfindung angewendet werden.

Die erfindungsgemässe Vorrichtung weist gegenüber den bestehenden Einrichtungen eine Anzahl von Vorteilen auf. So fixiert das Teilverhältnis und die Pulsfrequenz des Hochfrequenzoszillators den Frequenzunterschied, so dass keine zusätzliche Wartung, Abstimmung oder Bedienung notwendig ist. Die Interferenzfrequenz hängt daher weder von Temperaturunterschieden, Spannungsschwankungen, noch von anderen Faktoren ab, so dass sich ein einfacher und billiger Aufbau ergibt. Ein wirtschaftlicher Vorteil ist der einfache Aufbau aus integrierten Bauteilen, so dass es möglich ist, diese Anordnung nicht nur wirtschaftlich, sondern auch klein und handlich zu gestalten. Schliesslich ermöglicht der digitale Aufbau der Anordnung den Einsatz von Sensorschaltungen und Zeitschalteinrichtungen und damit eine Vollautomatisierung der Interferenzstromeinrichtung.

Weitere Vorteile und Merkmale der Erfindung ergeben sich aus den Ansprüchen und aus der nachfolgenden Beschreibung, in der Ausführungsbeispiele anhand der Zeichnung im einzelnen erläutert sind. Dabei zeigt:

Fig. 1 ein Blockschema mit zugehörigem Impulsschema;

Fig. 2 eine Vorrichtung mit einer programmierbaren Frequenzteilerstufe;

Fig. 3 eine Ausführungsform, bei der durch Aussperrung einzelner Taktimpulse die Frequenz der einen Teilfrequenz geändert wird;

Fig. 4 das Impulsdiagramm hierzu;

Fig. 5 eine Ausgestaltung, bei der durch Hinzufügen von phasenverschobenen Taktimpulsen die Frequenz des einen Teilkreises geändert wird; und

Fig. 6 eine weitere Ausführungsform zur Frequenzteilung mit einem Komparator.

Ein gemeinsamer Hochfrequenzoszillator 1 erzeugt eine hohe Taktfrequenz 15. Diese wird sowohl einem fest eingestellten Frequenzteiler 3 als auch einem veränderlichen Frequenzteiler 2 zugeführt. Bei Verwendung eines dekadischen Teilers entstehen schmale Ausgangsimpulse 16 am Frequenzteiler, die in je einer Impulsformerstufe 4 in eine symmetrische Rechteckwelle 17 umgewandet werden. Jeweils eine Kurvenformstufe 5 verwandelt die Rechteckwelle 17 in eine Sinuswelle 18. Die Kurvenformstufe 5 besteht im Prinzip aus einem Grundwellenfilter. Die Leistungsverstärker 6 führen über zwei getrennte Stromkreise die Teilströme dem Patienten 7 zu, in dessen Körper die Überlagerung erfolgt und damit die Interferenz entsteht. Ein zusätzlicher Frequenzteiler 8, der durch eine Teilerumschalteinrichtung 9 umgestellt werden kann, setzt die fest eingestellte Ausgangsfrequenz des Frequenzteilers 3 auf eine Frequenz von ca. 1 Hz herab am Ausgang von 8 und schaltet so eine Steuerschaltung 10 des veränderlichen Frequenzteilers 2 alle Sekunden um, so dass sich im Sekundenrhythmus die Interferenz automatisch ändert. Diese Automatik ist mittels eines Schalters abschaltbar, so dass die Teilerumschalteinrichtung 9 auch willkürlich verändert werden kann. Dann liefert diese Vorrichtung also fest einstellbare Interferenzerscheinungen, die der Interferenzstrommethode in der heutigen Praxis entsprechen. Liefert der Hochfrequenzoszillator z.B. die Taktfrequenz 15 in der Grösse von 10 MHz, so wird bei 2000facher Teilung an den Ausgängen der Frequenzteiler 2 und 3 ein Signal mit einer Frequenz von 5000 Hz entstehen. Am Ausgang der Impulsformerstufen 4 entstehen Rechteckimpulse 17 mit 2500 Hz, die in sinusförmige Signale umgewandelt werden und im Körper 7 die Interferenzfrequenz Null ergeben. Ändert sich nun das Teilungsverhältnis vom Frequenzteiler 2, dann ändert sich die Frequenz des Signals 17 nach der Impulsformerstufe 4, beispielsweise auf 2501,25 Hz, wenn das Frequenzteilerverhältnis aus 1999 geändert wurde, da die Impulsformerstufe 4 die Frequenz noch einmal durch 2 teilt. Die im Körper 7 entstehende Interferenzfrequenz ist in diesem Falle 1,25 Hz, und die dem Körper zugeführte Mittelfrequenz 2500 und 2501,25 Hz.

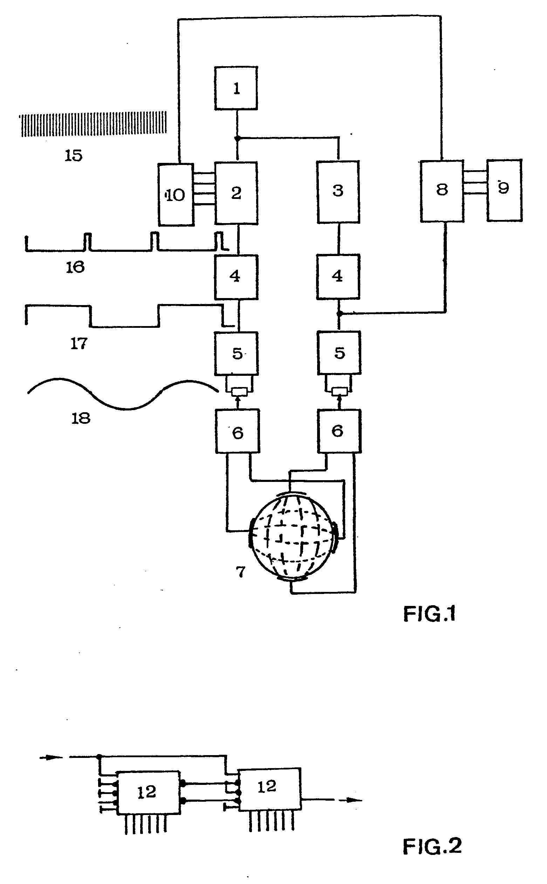

Figur 2 zeigt das Schaltbild eines programmierbaren Frequenzteilers, der aus zwei Zählerbausteinen 12 aufgebaut ist. Eine derartige Frequenzteilerschaltung kann ihr Teilungsverhältnis von 1:4096 bis zu 4095:4096 ändern. Die Zählerbausteine 12 sind handelsübliche Schaltkreise, die keiner zusätzlichen Beschaltung bedürfen. Diese Schaltkreise sind durch interne logische Verknüpfungen und Programmausgänge dermassen aufgebaut, dass beliebige, auch ungradzahlige Teilungsverhältnisse erzielbar sind.

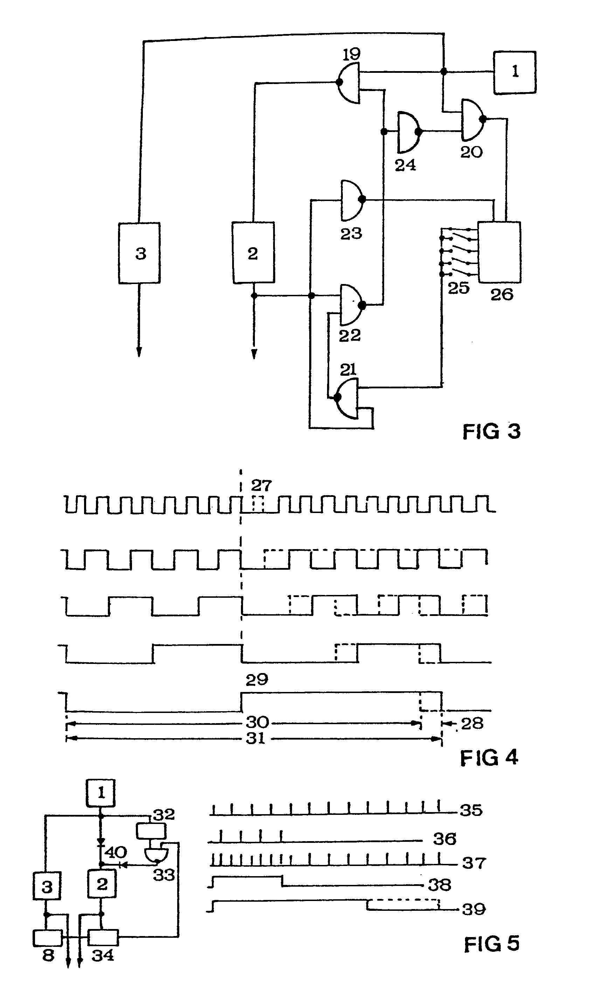

Eine andere Möglichkeit, die Frequenzteilung zu ändern, zeigt Fig. 3. Der Hochfrequenzoszillator 1 erzeugt eine Rechteckwelle von 25 MHz, welche über ein als elektronisch schaltbare Sperrvorrichtung dienendes NAND-Gatter 19 den Binär-Frequenzteiler 2 taktet, und zwar solange, bis der Ausgang des Frequenzteilers 2 von

Null auf L schaltet. Durch dieses L wird ein NAND-Gatter 22 von L auf Null gestellt und hierdurch das NAND-Gatter 19 gesperrt und damit der Takt des Frequenzteilers 2 gesperrt. Der Übergang am Ausgang 2 des Teilers von Null auf L stellt ausserdem über einen Inverter 23 den Resteingang eines Zählers 26 von L auf Null und weiterhin einen Eingang eines NAND-Gatters 21 auf L und bereitet damit das NAND-Gatter 21 zur Umschaltung vor. Dann wird durch die Null am Ausgang des NAND-Gatters 22 über einen Inverter 24 ein NAND-Gatter 20 leitend und damit der Zähler 26 angesteuert, bis dessen Ausgang von Null auf L schaltet. Dies bewirkt, dass der Ausgang des NAND-Gatters 21 von L auf Null schaltet, damit ebenfalls das NAND-Gatter 22 von Null auf L schaltet und über den Inverter 24 das NAND-Gatter 20 sperrt und den Zähler 26 stoppt. Gleichzeitig wird das NAND-Gatter 19 durchgeschaltet, und der Frequenzteiler 2 arbeitet weiter. Wenn der Ausgang vom Frequenzteiler 2 von L auf Null schaltet, wird über den Inverter 23 der Reseteingang vom Zähler 26 auf L gestellt und damit bewirkt, dass der Zähler 26 auf Null zurückgeht und der Ausgang auf Null steht. Hierdurch wird der Ausgang des NAND-Gatters 21 auf L gestellt und damit das NAND-Gatter 22 zur Umschaltung beim nächsten Wechsel von Null auf L des Frequenzteilers 2 vorbereitet. Diese Frequenzteilerschaltung bewirkt, dass durch Auslassen von einzelnen oder mehreren Impulsen pro Ausgangsperiode die Ausgangsperiode verlängert wird. Die Anzahl der ausgelassenen Impulse und damit die Periodenverlängerung am Ausgang wird durch den Zähler 26, dessen Ausgänge durch einen Schalter 25 entsprechend der gewünschten Verlängerung an das NAND-Gatter 21 angeschlossen. Das Auslassen eines Impulses bei 25 MHz Taktfrequenz und 5000facher Teilung ergibt eine Frequenzänderung am Ausgang des Frequenzteilers 2 von 5000 auf 4999 Hz. Dies entspricht einer Frequenzdifferenz zwischen Frequenzteiler 3 und Frequenzteiler 2 und damit einer Interferenzfrequenz von 1 Hz.

Fig. 4 zeigt ein Impulsdiagramm in vereinfachter Darstellung für eine 16fache Teilung. Für 5000fache Frequenzteilung gilt dasselbe Prinzip. 15 sind die Taktimpulse des Hochfrequenzoszillators 1, von denen beim Umschalten der Ausgangsperiode 29 des Frequenzteilers 2 von Null auf L der folgende Taktimpuls 27 gesperrt wird. Hierdurch ergibt sich der Ausfall einer Taktperiode 28 auf allen folgenden Teilerstufen und damit eine Verlängerung der Ausgangsperiode von 30 auf 31, wodurch in diesen gezeichneten Kurvenabschnitten das Frequenzteilerverhältnis von 16 auf 17 erhöht wird.

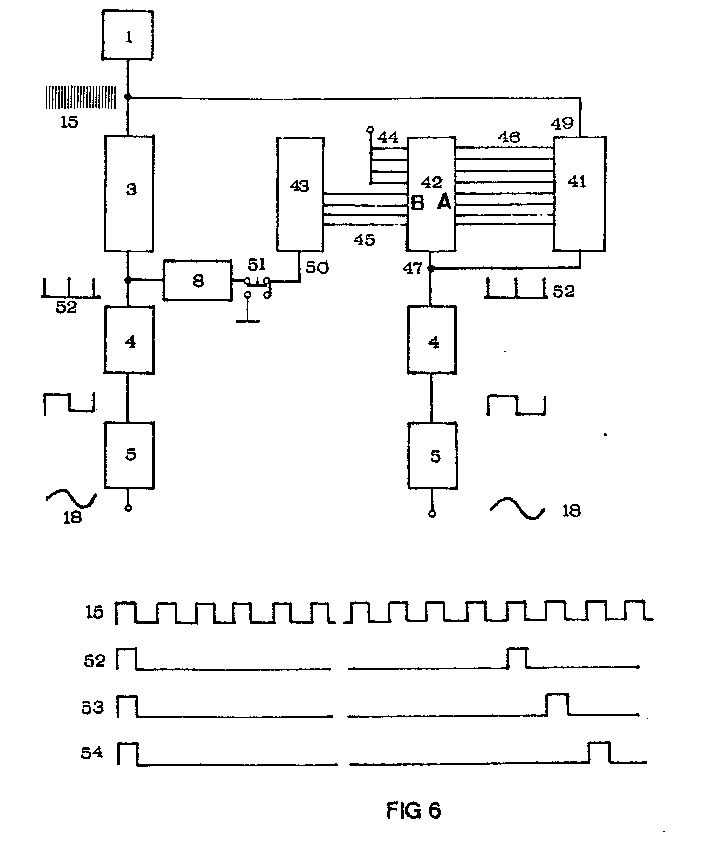

In Fig. 5 ist 1 ebenfalls der Hochfrequenzoszillator mit 25 MHz, dessen hohe Taktfrequenz 35 einmal dem fest eingestellten Frequenzteiler 3, einmal über eine Modulatorschaltung 33, 40 dem veränderlichen Frequenzteiler 2 und einmal einem Phasenschieber 32 zugeführt wird. Im Phasenschieber 32 wird die Phasenlage der Taktimpulse derart verändert, dass die über den Teilkreis der Modulatorschaltung dem NAND-Gatter 33 geschalteten Impulse 36 über die Dioden 40 am Eingang des Frequenzteilers 2 zwischen den direkt vom Hochfrequenzoszillator 1 kommenden Taktimpulsen liegen (Kurvenbild 37). Der Ausgang des Frequenzteilers 2 startet einen monostabilen Multivibrator 34, dessen Impulsbreite 38 automatisch durch den Frequenzteiler 8, der die Ausgangsfrequenz des Frequenzteilers 3 auf ca. 1 Sekunde herabsetzt, festgelegt wird. Während der Impulsdauer 38 am Multivibrator 34 ist das NAND-Gatter leitend, und die Diodenmischschaltung 40 setzt die Taktimpulse 35 mit den phasenverschobenen Impulsen 36 zusammen. Hierdurch zählt der Frequenzteiler 2 in der Zeitdauer des Impulses 38 im 50 MHz-Takt und verkürzt damit die Ausgangsperiode 39. Bei Hinzufügen eines einzelnen Impulses ergibt sich eine Frequenzerhöhung bei 5000facher Teilung von 5000 und 5000,5. Daraus ergibt sich die kleinste Interferenzfrequenz von 0,5 Hz. Durch den zusätzlichen Frequenzteiler 8, der dem fest eingestellten Frequenzteiler 3 nachgeschaltet ist, kann eine Steuerspannung zur automatischen Steuerung des Multivibrators 34 gewonnen werden. Diese Steuerspannung verändert hierbei die Impulsbreite des monostabilen Multivibrators, z.B. durch Integrierung der am Ausgang des Frequenzteilers 8 auftretenden Frequenz (in der Abbildung nicht dargestellt). Durch diese Integration bildet sich eine Dreieckspannung, die zur Impulsbreitenmodulation eines monostabilen Multivibrators benutzt werden kann.

In Fig. 6 besteht die Vorrichtung zur Frequenzänderung aus einem Zähler 41, dessen Takteingang 49 mit dem Hochfrequenzoszillator 1 verbunden ist. Die Ausgänge 46 des Zählers 41 sind mit Vergleichseingängen A eines Digitalkomparators 42 und einem Teil der Vergleichseingänge B mit den Ausgängen 45 eines Vor-Rückwärtszählers 43, dessen Takteingang 50 durch Einzelimpulse automatisch getaktet wird, verbunden. Ein Teil von Vergleichseingängen B des Komparators 42 ist hierbei mit einer logischen «1» 44 verbunden. Ein Ausgang 47 A–B des Komparators hingegen ist zur Abnahme der geregelten Frequenz mit einer Impulsformerstufe zusammengeschaltet und ausserdem mit dem Reseteingang des Zählers 41 verbunden. Der Zähler 43 kann hierbei durch einen Umschalter 51 resp. vorzugsweise durch einen weiteren Teiler 8, der die Teilfrequenz des zweiten Teilers 3 weiter auf einen Ausgangstakt von ca. 1 Sekunde herabsetzt, takten, so dass sich die Frequenz alle Sekunden ändert. 5 sind die Kurvenformerstufen zum Umwandeln der Rechteck- in Sinuskurven. Wenn 15 die Oszillatorfrequenz als Taktfrequenz ist, dann ist – bedingt durch die festen mit einer logischen «1» 44 beschalteten Eingänge B – der Kurvenzug 52 die Ausgangsfrequenz am Komparator 42. Wenn der Zähler 43 auf seiner ersten Stellung steht, ist 53 die Ausgangsfrequenz dieses Komparators. 54 ist die Ausgangsfrequenz, wenn der Zähler 43 auf Stellung 2 steht usw. Die Ausgangsperiode wird also durch jede Zähler- änderung um eine Taktperiode länger. Durch die Pulsformerstufe 4 wird die Ausgangsfrequenz eine symmetrische Rechteckwelle und durch die Kurvenformerstufe 5 in eine Sinuswelle 18 umgewandelt.

Es gehört zum Erfindungsgedanken, dass die Ansteuerung des Monoflops des Multivibrators oder des Zählers zur Frequenzänderung auch mit anderen Parametern als beschrieben, wie Dreieckspannung, Rechteckspannung oder durch von linearen Bauelementen erzeugte Kurvenformen durchgeführt werden kann, wobei in jedem der Beispiele die Zähl- und Umschalteinrichtung auch durch einen Monoflop oder monostabilen Multivibrator oder eine andere Schalteinheit, die in der Lage ist, in der Impulsbreite steuerbare Signale zu liefern, erfolgen kann.

## Patentansprüche

1. Vorrichtung zur Interferenzstromtherapie, mit einem gemeinsamen Oszillator für beide vorhandenen Stromkreise, dadurch gekennzeichnet, dass der Oszillator ein Hochfrequenzoszillator (1) ist; dass der Hochfrequenzoszillator (1) mit den Eingängen mindestens zweier digitaler Frequenzteiler (2, 3) verbunden ist; dass zumindest an einem Frequenzteiler (2) eine digitale Rückkopplungseinrichtung (8, 9, 10; 19; 32, 33, 40, 41, 42, 43) zum Einstellen eines geringen Frequenzunterschiedes der an den Ausgängen der Frequenzteiler (2, 3) gegebenen Behandlungsströme vorgesehen ist.

2. Vorrichtung nach Anspruch 1, dadurch gekennzeichnet, dass die digitale Rückkopplungseinrichtung aus einer zwischen den Hochfrequenzoszillator (1) und den Eingang des Frequenzteilers (2) geschalteten, elektronisch schaltbaren Sperrvorrichtung (19), die mittels einer Steuereinrichtung (20, 21, 22, 23, 24 und 26) mit dem Ausgang des Frequenzteilers (2) verbunden ist, besteht und pro Ausgangsperiode (30) eine Anzahl Taktimpulse (27) aussperrt und damit die Ausgangsfrequenz des Frequenzteilers (2) erniedrigt.

3. Vorrichtung nach Anspruch 1, dadurch gekennzeichnet, dass die digitale Rückkopplungseinrichtung aus einer Modulatorschaltung (33, 40) besteht, die zwischen dem Hochfrequenzoszillator (1) mittels eines Phasenschiebers (32) und dem Eingang des Frequenzteilers (2) geschaltet ist, sowie dass eine Steuerschaltung (34) mit dem Ausgang des Frequenzteilers (2) verbunden ist, die bewirkt, dass pro Ausgangsperiode (39) in der durch die Steuerschaltung (34) festgelegten Zeit (38) phasenverschobene Impulse (36) zwischen den Hochfrequenzoszillatorimpulsen (35) hinzugefügt werden und damit die Ausgangsfrequenz am Frequenzteiler (2) erhöht wird.

4. Vorrichtung nach Anspruch 3, dadurch gekennzeichnet, dass die Steuerschaltung (34) aus einem monostabilen Multivibrator mit steuerbarer Impulsbreite besteht.

5. Vorrichtung nach einem der vorangehenden Ansprüche, dadurch gekennzeichnet, dass einem Frequenzteiler (3) ein weiterer Frequenzteiler (8) nachgeschaltet ist, durch dessen mit Hilfe eines Umschalters (9) erzeugten Ausgangssignals über eine Steuerschaltung (10) des Teilerverhältnis des veränderbaren Frequenzteilers (2) automatisch kontinuierlich veränderbar ist.

6. Vorrichtung nach einem der vorangehenden Ansprüche, dadurch gekennzeichnet, dass jedem Frequenzteiler (2, 3) zur Umwandlung der Rechteckkurve (17) in eine Sinuskurve (18) eine Kurvenformstufe (5) nachgeschaltet ist.

7. Vorrichtung nach Anspruch 1, dadurch gekennzeichnet, dass die digitale Rückkopplungseinrichtung einen Zähler (41) aufweist, dessen Takteingang (49) mit dem Hochfrequenzoszillator (1) verbunden ist, während die Ausgänge des Zählers (41) hingegen mit Vergleichseingängen A eines Digitalkomparators und ein Teil der Vergleichseingänge B mit den Ausgängen eines Vor-Rückwärtszählers (43) verbunden sind, dessen Taktfrequenz durch Einzelimpulse automatisch getaktet wird; dass ein Teil der Vergleichseingänge B des Komparators mit einer logischen «1» (44) verbunden ist, wobei der Ausgang A–B des Komparators zur Abnahme der geregelten Frequenz mit einer Impulsformerstufe (4) und dem Resteingang des Zählers (41) verbunden ist.

8. Vorrichtung nach einem der vorangehenden Ansprüche, dadurch gekennzeichnet, dass der Hochfrequenzoszillator (1), die Frequenzteiler (2, 3) und die digitale Rückkopplungseinrichtung (8, 9, 10, 19, 32, 33, 40, 41, 42 und 43) einen entsprechenden Mikroprozessor aufweisen, der im Sinne der Funktionsabläufe programmierbar ist.

## Revendications

1. Appareil de thérapie par courants d'interférence, comprenant un oscillateur commun pour les deux circuits de courant disponibles, caractérisé en ce que l'oscillateur est un oscillateur haute-fréquence (1), en ce que l'oscillateur haute-fréquence (1) est relié aux entrées d'au moins deux diviseurs de fréquence (2, 3) numériques, et en ce que, à au moins un diviseur de fréquence (2) est associé un dispositif (8, 9, 10, 19, 32, 33, 40, 41, 42, 43) numérique de couplage rétroactif pour établir une faible différence entre les fréquences des courants de traitement fournis aux sorties des diviseurs de fréquence (2, 3).

2. Appareil selon la revendication 1, caractérisé en ce que le dispositif numérique de couplage rétroactif consiste en un dispositif de blocage (19), commutable électroniquement et monté entre l'oscillateur haute-fréquence (1) et l'entrée du diviseur de fréquence (2), qui est relié a la sortie du diviseur de fréquence (2) au moyen d'un dispositif de commande (20, 21, 22, 23, 24 et 26), et qui bloque un certain nombre d'impulsions de cadence ou d'horloge (27) par période de sortie (30) et ainsi abaisse la fréquence de sortie du diviseur de fréquence (2).

3. Appareil selon la revendication 1, caractérisé en ce que le dispositif de couplage rétroactif consiste en un circuit modulateur (33, 40) qui est monté entre l'oscillateur haute-fréquence (1), grâce à un déphaseur (32), et l'entrée du diviseur de fréquence (2), et en ce qu'un circuit de commande (34) est relié à la sortie du diviseur de fréquence (2) pour assurer que, pour chaque période de sortie (39), des impulsions déphasées (36) soient introduites entre les impulsions (35) de l'oscillateur haute-fréquence, dans le temps (38) fixé par le circuit de commande (34), la fréquence de sortie du diviseur de fréquence (2) étant ainsi augmentée.

4. Appareil selon la revendication 3, caractérisé en ce que le circuit de commande (34) consiste en un multivibrateur monostable à largeur d'impulsion réglable.

5. Appareil selon l'une quelconque des revendications précédentes, caractérisé en ce qu'à un diviseur de fréquence (3) fait suite un autre diviseur de fréquence (8) dont le signal de sortie produit à l'aide d'un commutateur (9) permet de faire varier automatiquement en continu le rapport du diviseur fréquence (2) réglable, au moyen d'un circuit de commande (10).

6. Appareil selon l'une quelconque des revendications précédentes, caractérisé en ce qu'un étage (5) de conversion curviligne fait suite à chaque diviseur de fréquence (2, 3) pour transformer la courbe rectangulaire (17) en une courbe sinusoïdale (18).

7. Appareil selon la revendication 1, caractérisé en ce que le dispositif numérique de couplage rétroactif comprend un compteur (41) dont l'entrée de cadence ou d'horloge (49) est reliée à l'oscillateur haute-fréquence (1), alors que les sorties du compteur (41) sont par contre reliées aux entrées de comparaison A d'un comparateur numérique et qu'une partie des entrées de comparaison B sont reliées aux sorties d'un compteur-décompteur (43), dont la fréquence de cadence est cadencée automatiquement par des impulsions isolées, et en ce qu'une partie des entrées de comparaison B du comparateur est reliée à un «1» logique (44), la sortie A–B du comparateur, pour la réception de la fréquence réglée, étant reliée à un étage (4) de mise en forme d'impulsion et à l'entrée de remise à zéro du compteur (41).

8. Appareil selon l'une quelconque des revendications précédentes, caractérisé en ce que l'oscillateur haute-fréquence (1), les diviseurs de fréquence (2, 3) et le dispositif numérique de couplage rétroactif (8, 9, 10, 19, 32, 33, 40, 41, 42 et 43) sont équipés d'un microprocesseur correspondant qui peut être programmé dans le sens du déroulement fonctionnel.

**Claims**

1. Apparatus for interference current therapy with a common oscillator for both the circuits present, characterized in that the oscillator is a radio-frequency oscillator (1), that the radiofrequency oscillator (1) is connected to the inputs of at least two digital frequency dividers (2, 3) and that on at least one frequency divider (2) is provided a digital feedback device (8, 9, 10; 19; 32, 33, 40, 41, 42, 43) for setting a limited frequency difference of the treatment currents at the outputs of the frequency divider (2, 3).

2. Apparatus according to claim 1, characterized in that the digital feedback device comprises an electronically switchable blocking device (19) connected between the radiofrequency oscillator (1) and the input of the frequency divider (2), said device (19) being connected by means of a control device (20, 21, 22, 23, 24, 26) to the output of frequency divider (2) and a plurality of timing pulses (27) is blocked out during each output cycle (30) and as a result the output frequency of frequency divider (2) is reduced.

3. Apparatus according to claim 1, characterized in that the digital feedback device comprises a modulation circuit (33, 40) connected between the radio-frequency oscillator (1) by means of a phase shifter (32) and the input of frequency divider (2), whilst a control circuit (34) is connected to the output of frequency divider (2) and as a result in the time (38) fixed by control circuit (34), dephased pulses (36) are added between the radiofrequency oscillator pulses (35) during each output cycle (39) and consequently the output frequency at the frequency divider (2) is raised.

4. Apparatus according to claim 3, characterized in that the control circuit (34) comprises a monostable mutlivibrator with a controllable pulse width.

5. Apparatus according to one of the preceding claims, characterized in that a further frequency divider (8) is connected in series with a frequency divider (3), the division ratio of the variable frequency divider (2) being automatically continuously variable by means of a control switch (10) through the output signal of frequency divider (8) produced with the aid of a changeover switch (9).

6. Apparatus according to one of the preceding claims, characterized in that a curve shape stage (5) is connected in series with each frequency divider (2, 3) for converting the square-wave curve (17) into a sine curve (18).

7. Apparatus according to claim 1, characterized in that the digital feedback device has a counter (41), whose timing input (49) is connected to the radio-frequency oscillator (1), whilst the outputs of counter (41) are connected to the reference inputs A of a digital comparator and part of the reference inputs B with the outputs of a bidirectional counter (43) whose timing frequency is automatically timed by single-shot pulses and that part of the reference inputs B of the comparator is connected to a logic «1» (44), output A–B of the comparator being connected to a pulse shaper stage (4) and the resetting input of counter (41) for accepting the control frequency.

8. Apparatus according to one of the preceding claims, characterized in that the radiofrequency oscillator (1), the frequency divider (2, 3) and the digital feedback device (8, 9, 10, 19, 32, 33, 40, 41, 42 and 43) have a corresponding microprocessor, programmable in the sense of the functional sequences.

FIG.1

FIG.2

FIG 3

FIG 4

FIG 5

FIG 6